# EUROPEAN PATENT APPLICATION

(11) **EP 1 669 083 A1**
(43) Date of publication of application: **14.06.2006**
(21) Application number: 04078353.2
(22) Date of filing: 10.12.2004
(51) Int. Cl.: A61K 38/02, A61K 48/00, A61P 9/00

(54) **Heat shock proteins (HSP) and supraventricular arrhythmia**

(71) Applicant: Zernike Business Support B.V., 9747 AN Groningen (NL)
(72) Inventor: Henning, Robert Henk, 9919 AJ Loppersum (NL); Kampinga, Harm Harmannus, 9791 KG Ten Boer (NL); Brundel, Bianca Johanna Josephina Maria, 9737 ST Gronigen (NL)
(74) Representative: Winckels, Johannes Hubertus F.

(57) **Abstract**

The invention relates to the field of biology, molecular biology and medicine. More specifically, the invention relates to a method for at least in part preventing or delaying or decreasing damage to a cardiac cell induced by a supraventricular arrhythmia. The invention provides a method for preventing, delaying or decreasing damage to a cardiac cell induced by a supraventricular arrhythmia comprising increasing the amount of at least one heat shock protein (HSP) or a functional equivalent and/or a functional fragment thereof, e.g. HSP27 or its functional equivalent HSP25, in said cardiac cell.

## Description

The invention relates to the field of biology, molecular biology and medicine. More specifically, the invention relates to a method for at least in part preventing or delaying or decreasing damage to a cardiac cell wherein said damage is induced by a supraventricular arrhythmia.

Atrial fibrillation (AF) is the most common cardiac arrhythmia which has the tendency to become more persistent over time.¹ Recent research exploring the underlying mechanisms of the self-perpetuation of AF has demonstrated the high rate of myocyte activation during AF to induce primarily myocyte stress, which in turn leads to heterogeneity of the electrical activation pattern ²⁻¹⁰ and loss of contractile function.¹¹⁻¹⁵ When the arrhythmia continues, AF induces changes at the structural level, predominantly myolysis, which are of prime importance for contractile dysfunction and vulnerability of AF.^{6;12;16-19}

Myolysis is characterized by disruption of the myofibril structure ^{12;13;20} and observed after various forms of cell stress such as ischemic stress²¹ and hypoxia.²² Myocytes turn into a non-functional phenotype, by disruption of the myofibril structure which leads to myolysis and as a consequence to contractile dysfunction.

It is a goal of the present invention to develop methods and pharmaceutical compositions for preventing, delaying or decreasing a deteriorating/negative effect on a cardiac cell said effect being induced by a supraventricular arrhythmia, such as AF.

The present inventors now disclose for the first time that an increased expression of heat shock protein 27 (HSP27; in rodents often referred to as HSP25) and heat shock protein 70 (HSP70) is present in patients with paroxysmal AF. We subsequently extended our study to a paced cell model for AF.²⁸ The present invention discloses that induction of HSP, in particular HSP27, attenuates pacing-induced myolysis.

Thus in a first embodiment the invention provides a method for at least in part preventing, delaying or decreasing damage to a cardiac cell induced by a supraventricular arrhythmia comprising increasing the amount of at least one heat shock protein (HSP) or a functional equivalent and/or a functional fragment thereof in said cardiac cell.

Heat shock proteins (HSPs) represent a group of chaperones. Major classes of HSPs in cardiovascular biology are HSP110, HSP90, HSP70, small HSP (such as HSP27), assorted (such as HSP47 or HSP40) and HSP60. Some of these HSPs have been tested for their clinical relevance in conditions such as cardiac hypertrophy, vascular wall injury and ischemic preconditioning. A substantial amount of literature describes the induction of HSP70 by ischemia, the potential role of HSP70 in ischemic preconditioning, and an inverse correlation between expression of HSP70 induced by ischemic or thermal preconditioning and infarct size in animal models. The focus in these publications is on ventricular conditions and HSPs.

A supraventricular arrytmia is defined herein as an arrhythmia that originates from above the ventricles. "Supra" means above and "ventricular" refers to the lower chambers of the heart (ventricles).

Preferably, a method according to the invention results in at least in part preventing, delaying or decreasing damage to a cardiac cell. Prevention is possible when no (visible) damage to a cardiac cell has occurred yet. In this case, by providing HSP to such a cell, damage (for example myolysis) is preferably completely inhibited. Decreasing is possible when a cardiac cell already has some (visible) damage as induced by a supraventricular arrhytmia. In this case the (visible) damage is reduced, preferably completely abolished. Delaying is possible when damage is already or is not present. Preferably, the delaying is such that (further) (visible) damage is postponed as long as possible.

A fragment of an HSP protein is herein defined as a fragment of an HSP molecule which fragment comprises a deletion at the N-terminus or at the C-terminus or of an internal part of an HSP protein or any combination of these possibilities. The fragment must however be functional, i.e. it must be capable of preventing, delaying or decreasing damage to a cardiac cell, said damage being induced by a supraventricular arrhythmia. An equivalent is herein defined as a mutant HSP of which the amino acid sequence has been altered/mutated in such a way that the resulting HSP comprises mutations (insertions, point mutations) compared to the original HSP, but again such mutants must be functional i.e. it must be capable of preventing, delaying or decreasing damage to a cardiac cell, said damage being induced by a supraventricular arrhythmia. Moreover, the term functional equivalent also includes HSPs from other origins, i.e. HSP27 (from human origin) is a functional equivalent of HSP25 (from murine origin) or the other way around. Moreover, the properties of a functional fragment and/or a functional equivalent are the same in kind, not necessarily in amount. To avoid activation of the immune system (for example antibody formation) it is preferred to use a species specific HSP in a treatment. If for example HSP is injected during an operation in a human heart the HSP is preferably of human origin or is humanised or a human gene encoding HSP is expressed in an expression system that allows for proper expression/processing. If for example a mouse is treated with help of gene delivery therapy the provided HSP gene is preferably of murine origin or is adapted to express a non-immunogenic HSP.

In a preferred embodiment the invention provides a method for at least in part preventing, delaying or decreasing damage to a cardiac cell induced by a supraventricular arrhythmia comprising increasing the amount of at least one heat shock protein (HSP) or a functional equivalent and/or a functional fragment thereof in said cardiac cell, wherein said HSP is HSP27 or an HSP27-like protein or a functional equivalent and/or a functional fragment thereof. As disclosed herein within the experimental part over-expression of HSP27 leads to protection from pacing-induced myolysis. An example of an HSP27-like protein is HSP25. Again, to avoid activation of the immune system (for example antibody formation) it is preferred to use a species specific HSP in a treatment. In this case the HSP25 is preferably humanised when applied to humans.

As disclosed herein within the experimental part, there are different ways in which the amount of at least one heat shock protein (HSP) or a functional equivalent and/or a functional fragment thereof may be increased in a cardiac cell. In a preferred embodiment the invention provides a method for at least in part preventing, delaying or decreasing damage to a cardiac cell induced by a supraventricular arrhythmia comprising increasing the amount of at least one heat shock protein (HSP) or a functional equivalent and/or a functional fragment thereof in said cardiac cell, wherein said HSP is increased in said cell by transfecting said cell with a gene encoding said HSP or a functional equivalent and/or a functional fragment thereof. The transfection may be transient as well as (more) permanent, for example by delivering the necessary genetic information to a bone marrow cell. In another preferred embodiment the amount of HSP is increased in said cell by injecting into said cell an HSP protein or a functional equivalent and/or a functional fragment thereof. In yet another preferred embodiment the amount of HSP is increased in said cell by providing said cell with a drug capable of increasing the amount of HSP. An increase of the amount of HSP may also be accomplished by heat preconditioning of the relevant cell. This is for example performed to, at least in part, prevent (post-)operative AF as regularly seen at open-heart surgery. It is clear that the choice of how to increase the HSP amount depends on the circumstances, for example on whether the method is applied in vivo or in *vitro.*

In yet another embodiment the invention provides a method for at least in part preventing, delaying or decreasing damage to a cardiac cell induced by a supraventricular arrhythmia comprising increasing the amount of at least one heat shock protein (HSP) or a functional equivalent and/or a functional fragment thereof in said cardiac cell, wherein said supraventricular arrhythmia is atrial fibrillation (AF). The present invention shows that upregulation of HSP represents a therapeutic goal to prevent or delay the self-perpetuation/progression of AF. Other examples of supraventricular arrhythmias are Atrial flutter, AV nodal re-entry tachycardias or tachycardia due to an accessory pathway e.g. Wolf-Parkinson-White syndrome.

The cardiac cell in which the HSP according to a method of the invention is increased is for example an endothelial cell, a smooth muscle cell or a fibroblast. In a preferred embodiment the invention provides a method for at least in part preventing, delaying or decreasing damage to a cardiac cell induced by a supraventricular arrhythmia comprising increasing the amount of at least one heat shock protein (HSP) or a functional equivalent and/or a functional fragment thereof in said cardiac cell, wherein said cell is a cardiomyocyte. A method according to the invention is used for preventing, delaying or decreasing damage to a cardiac cell (or degeneration of a cardiac cell; the terms are used interchangeably herein) and preferably a method according to the invention is used for preventing or delaying or decreasing myocyte remodeling. Damage to or degeneration of a cardiac cell results in a deteriorating functioning of said cell compared to a cell not suffering from supraventricular arrhythmia. This deteriorating functioning of said cardiac cell leads for example to a less contractile capability of said cell. Preferably, applying a method according to the invention results in an adaptation and/or survival, i.e. remodeling, of said cell. Examples of myocyte remodeling are electrophysiological changes or changes in the protein expression profiles or a decrease in the amount of ion channels or a fast change in the function of ion channels or hibernation of a cardiac cell or contractile dysfunction of a cardiomyocyte. In a preferred embodiment said myocyte remodeling is myolysis. Myolysis is defined as the ability of myocytes to turn into a non functional phenotype, by disruption of the myofibril structure which leads to contractile dysfunction.

The method according to the invention can be applied in vivo as well as in vitro. In vivo the method is applied to non-human animal(s) (model systems) or to humans. The in vitro methods allow for fast screening of compounds which compounds are suspected to be capable of increasing the amount of HSP in a cardiac cell. For such an (high through put) in vitro test system, cells (for examples cardiomyocytes) are incubated with a (large) variety of possible effective compounds. After incubation with the compounds the proteins are extracted and the level of HSPs is determined by for example Western blotting and/or immunofluorescence. After selection of successful compounds/drugs, said drugs are tested in (smaller or larger) animal models.

In yet another embodiment, the invention provides a pharmaceutical composition comprising at least one nucleic acid encoding HSP or a functional equivalent and/or a functional fragment thereof and/or comprising at least one HSP protein or a functional equivalent and/or a functional fragment thereof and further comprising a pharmaceutical acceptable carrier or diluent. In a preferred embodiment, said HSP is HSP27 or an HSP27-like protein or a functional equivalent and/or a functional fragment thereof. In another preferred embodiment said pharmaceutical comprises multiple, for example at least two (or more), nucleic acids each encoding (possibly different) HSP or a functional equivalent and/or a functional fragment thereof (or one nucleic acid encoding two or more, possibly different HSPs). A pharmaceutical composition according to the invention that comprises at least one HSP protein or a functional equivalent and/or a functional fragment thereof is for example provided as a tablet or a fluid and is optionally protected for degradation by known, appropriate compositions. A pharmaceutical according to the invention may be provided by different routes of entrance, for example orally, rectally or by injection, nasally or by gene therapy.

In a preferred embodiment the pharmaceutical according to the invention comprises at least one HSP protein or a functional equivalent and/or a functional fragment thereof and forms part of a protein delivery system. In a preferred embodiment the invention provides a pharmaceutical composition comprising at least one nucleic acid encoding HSP or a functional equivalent and/or a functional fragment thereof and/or comprising at least one HSP protein or a functional equivalent and/or a functional fragment thereof and further comprising a pharmaceutical acceptable carrier or diluent, wherein said nucleic acid encoding HSP or a functional equivalent and/or a functional fragment thereof is part of a gene delivery vehicle. Gene delivery vehicles are well known to a person skilled in the art and hence no further elaboration is provided. Examples of gene delivery vehicle are adenovirus bases gene delivery systems or semliki forest virus based gene delivery vectors.

Said nucleic acid can be incorporated into the genome of said animal, and/or can be present transiently in said animal. Preferably transcription and/or translation of said nucleic acid is controlled by a signal, like for instance by a sequence responsive to exogenous compounds or responsive to increased stimulation of endogenous hormonal systems activated in cardiac disease, such as the RAS, natriuretic peptide system or the sympathetic system. Transcription and translation of said nucleic acid inside said animal results in the generation of HSP or a functional fragment and/or a functional equivalent thereof, which is for example capable of attenuating pacing-induced myolyis. As used herein, an animal can comprise a human and/or a non-human animal.

In one aspect of the invention, treatment involving HSP in a DNA based strategy comprises a treatment that is targeted to specific organs only, preferably the heart. In one embodiment of the invention, an HSP gene construct leads to conditional expression. The promoter of said construct reacts on the increase of neurohumoral levels indicative for a cardiac condition.

Of course, a person skilled in the art is well capable of choosing alternative ways for using HSP or a functional fragment and/or a functional equivalent thereof as a medicament for to prevent or delay the progression of a supraventricular arrhytmia, such as AF. Likewise, a person skilled in the art is well capable of performing alternative methods for using HSP or a functional fragment and/or a functional equivalent thereof for the preparation of a medicament.

Additives may be added to said medicament, for instance in order to facilitate administration and/or in order to enhance stability of said medicament.

As an example for optimisation of in *vivo* dosing of HSP inducers the following strategy is used. The first step comprises of defining the optimal time window of the experiments. To this extent the inducer will be administered via injection to the animal. The dose employed will be for example two-fold of those described in the literature or by the manufacturer (as described for other applications). Hearts will be removed at several time points following injection, e.g. 6, 12, 24 and 48 hrs. Induction of expression of HSPs will be studied by measurement of mRNA and/or protein levels of different HSPs. In a next series of experiments optimal dosing will be assessed using the optimal time window as determined previously. Animals will for example be injected with ¼ of the optimal dose described in the literature or by the manufacturer (as described for other applications). In each successive group of animals dosing will be doubled compared to the previous group. Analysis of induction will be performed as described for determination of the optimal time window.

In another embodiment, the invention provides the use of at least one gene encoding an HSP protein or a functional equivalent and/or a functional fragment thereof or at least one HSP protein or a functional equivalent and/or a functional fragment thereof for the (in vitro) treatment of a supraventricular arrhythmia.

In another embodiment, the invention provides the use of at least one nucleic acid encoding an HSP protein or a functional equivalent and/or a functional fragment thereof or at least one HSP protein or a functional equivalent and/or a functional fragment thereof for the manufacture of a medicament for the treatment of a supraventricular arrhythmia. In a preferred embodiment, said HSP is HSP27 or an HSP27-like protein or a functional equivalent and/or a functional fragment thereof. More preferred said nucleic acid encoding an HSP protein or a functional equivalent and/or a functional fragment thereof is part of a gene delivery vehicle. Even more preferred said supraventricular arrhythmia is atrial fibrillation. By treatment with such a medicament, myolysis in for example cardiomyocytes is at least in part prevented, delayed or decreased and hence the selfpertuation of AF is disrupted and (further) damage to a heart cell is prevented.

The methods and pharmaceutical compositions are for example used as a precautionary measure. For example, at surgery in general and specifically in open-heart surgery, a patient has a high risk of experiencing atrial fibrillation and as a consequence a patient is confronted with possible damage to a cardiac cell. In case a patient is treated prior and/or during and/or after surgery with a method according to the invention or treated with a pharmaceutical composition according to the invention the amount of HSP protein will be increased and the patient will not or suffer less from cardiac problems such a contractile dysfunction. During open-heart surgery it is fairly easy to inject HSP directly into the to be treated area or to provide the to be treated area with a gene encoding an HSP.

Yet another precautionary use of the method and/or a pharmaceutical composition according to the invention is preconditioning with HSP of a patient suffering from supraventricular arrhythmia to enhance success of cardioversion (for example with an on-demand pacemaker) to sinus rhythm. Successful cardioversion leads to a restoration of normal rhythm and atrial contractility, thus enabling discontinuation (or at least decreasing the amount) of anti-coagulation medicines. Consequently, patients are no longer at risk of side effects of anti coagulation medicines, i.e. risk of bleeding and in particular stroke.

The invention will be explained in more detail in the following description, which is not limiting the invention.

### EXPERIMENTAL PART

### MATERIALS AND METHODS

### Patients

Right and/or left atrial appendages (RAAs and LAAs respectively), as studied previously⁶, comprised of material from patients with PAF (n=8) or CAF (n=9) without additional underlying heart diseases and normal left ventricular function (Table 1). All AF patients underwent Maze surgery for difficult-to-treat AF. Presence, type and duration of AF were assessed based on the patient's history and previous electrocardiograms. As controls, appendages from patients with normal sinus rhythm undergoing coronary bypass grafting were used (CABG, n=8, Table 1). The Institutional Review Board approved the study and patients gave written informed consent.

### HL-1 cell culture conditions, transfections and constructs

The HL-1 atrial myocytes, developed from adult mouse atria²⁹ were obtained from Dr. William Claycomb (Louisiana State University, New Orleans, LA, USA) and cultured as described before.²⁸

Lipofectamine (Life technologies, The Netherlands) was used for transient transfections according to instructions of the manufacturer. pHSP70-YFP encodes a functional human HSP70 fused to YFP under control of a CMV promoter. pHSP27 encodes human HSP27 under control of CMV promoter.

### Pacing and induction of HSP expression

HL-1 myocytes (≥ 1x10⁶ myocytes) were cultured on coverslips and subjected to a 10-fold rate increase (rapid pacing) by electrical field stimulation (5 Hz, 1.5 V/cm field strength; Grass S88 stimulator).²⁸ Elevation of HSP expression in cultured myocytes was accomplished in 3 ways: (1) by subjection to a modest heat stress at 43°C for 30 min followed by overnight incubation at 37°C , and (2) by transfection of pHSP70-YFP or pHSP27 24hrs prior to pacing.

### Protein extraction and Western-Blot analysis

For Western-blot analysis, frozen RAAs and LAAs were used for protein isolation as described previously.⁶ For the isolation of proteins from HL-1 myocytes, the cells were lysed by the addition of SDS-PAGE sample buffer followed by sonication before separation on 10% PAA-SDS gels (1.10⁵ cells/slot). After transfer to nitrocellulose membranes (Stratagene, The Netherlands), membranes were incubated with primary antibodies against GAPDH (Affinity Reagents, USA), HSP25, HSP27, HSP40, Hsc70, HSP70 or HSP90 (all StressGen Biotechnologies, Victoria, Canada). Horseradish peroxidase-conjugated anti-mouse, anti-rat or anti-rabbit IgG (Santa-Cruz Biotechnology, The Netherlands) was used as secondary antibody. Signals were detected by the ECL-detection method (Amersham, The Netherlands) and quantified by densitometry. The amount of protein chosen was in the linear immunoreactive signal range and expressed relative to GAPDH.

### Immunofluorescent staining, quantification and confocal analysis

After subjecting HL-1 myocytes to rapid pacing, the cells were fixed for 10 minutes in 100% methanol (-20°C), dried and blocked in 5% BSA (20 minutes room temperature). Antibodies against myosin heavy chain (MF-20, Developmental Studies Hybridoma Bank, Baltimore, MD, USA) or HSP27 (StressGen Biotechnologies, Vicotria, Canada) were used as primary antibody. Fluorescein labeled isothiocyanate (FITC) anti-mouse and anti-rabbit (Jackson ImmunoResearch, The Netherlands) or N,N'-(dipropyl)-tetramethyl-indocarbocyanine Cy3 anti-mouse (Amersham, The Netherlands) were used as secondary antibody. Nuclei were visualized by 4',6-diamidino-2-phenylindole (DAPI) staining. Images of FITC, YFP or CY3 and DAPI fluorescence were obtained by using a Leica confocal laser-scanning microscope (Leica TCS SP2).

For the quantification of the amount of myolysis, at least 5 fields were examined with to a total amount of 250-500 myocytes, and myosin disruption (characteristic for myolysis¹²) was scored by three independent observers blinded for the experimental groups. Mean scores of the observers were used.

### Statistical Analysis

Results are expressed as mean ± SEM. All Western-Blot procedures and morphological quantifications were performed in duplo series of at least n=6 wells per series, and mean values were used for statistical analysis. The Mann-Whitney U-test was performed for group to group comparisons. All p-values were two-sided, a p-value of <0.05 was considered statistically significant. SPSS version 8.0 was used for all statistical evaluations.

### RESULTS

### HSP protein expression and structural changes in atrial tissue of patients with PAF and CAF

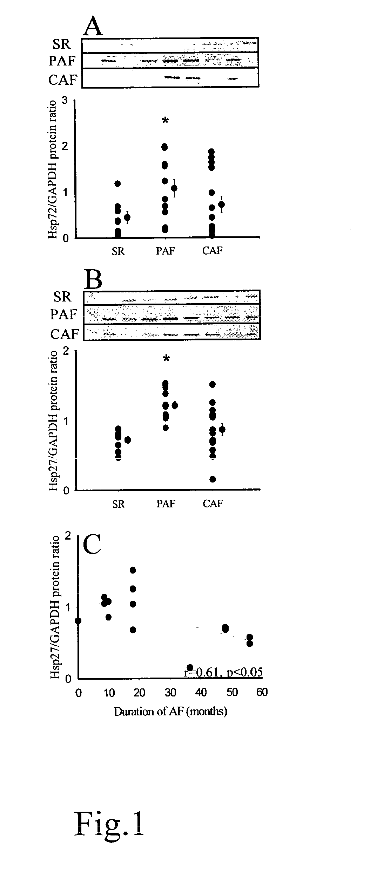

Proteins isolated from atrial appendages were used for immunological detection of HSP27, HSP40, Hsc70, HSP70 and HSP90. Changes in protein expression were studied in relation to protein levels of GAPDH, which did not differ between the groups (data not shown). Both the protein expression of HSP70 (Figure 1A) and of HSP27 (Figure 1B) were significantly increased in atrial tissue from patients with PAF compared to samples from control patients and patients with CAF. No significant changes in the amount of HSP40, Hsc70 and HSP90 were found (Table 1). Furthermore, HSP70 and HSP27 amounts in atrial tissue of CAF showed a large variation, which might be associated to the duration of the patient's arrhythmia. Therefore a correlation with the duration of CAF was made. Intriguingly, a significant inverse correlation was observed between the duration of CAF and HSP27 expression (Figure 1C). Patients with the shortest duration of AF revealed highest amount of HSP27 expression. No significant correlation between HSP27 expression and left atrial diameter, age, and medication as well HSP70 expression and CAF duration was observed (data not shown).

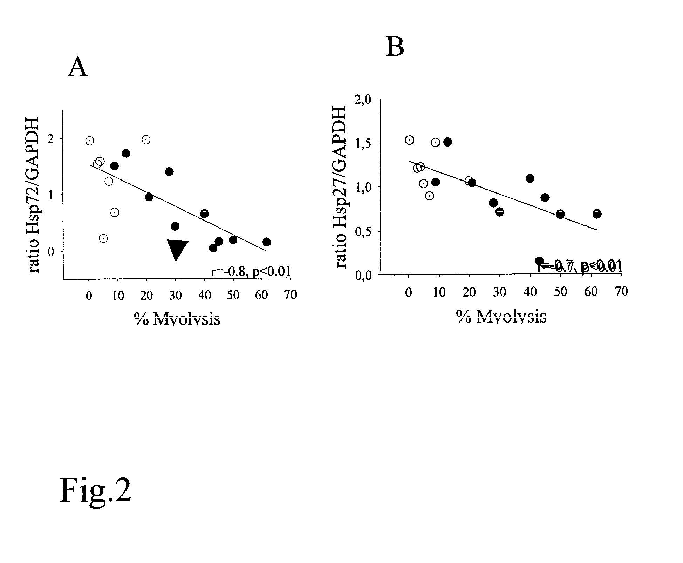

Previously we reported on (ultra)structural changes in atrial tissue of this patient population.¹¹ In brief, only in myocytes of patients with CAF a substantial fraction was myolytic (31.0 ± 14.8%), whereas the fraction of cells with myolysis in tissue of patients with PAF was low (6.9± 6.1%) and similar to that in control patients (5.5 ± 3.6%). An inverse correlation was found between the amount of myolysis and HSP70 and HSP27 expression in patients with AF (Figure 2A,B). Tissue of patients with increased HSP levels were associated with low amounts of myolysis. Confocal microscopy revealed that HSP27 was localized on myofibrils in cardiomyocytes whereas HSP70 showed diffuse cytosolic staining (not shown). These combined results indicate that increased levels of HSP in PAF patients convey a cytoprotective effect possibly linked to reduction of myolysis.

### HSP protect HL-1 myocytes from myolysis

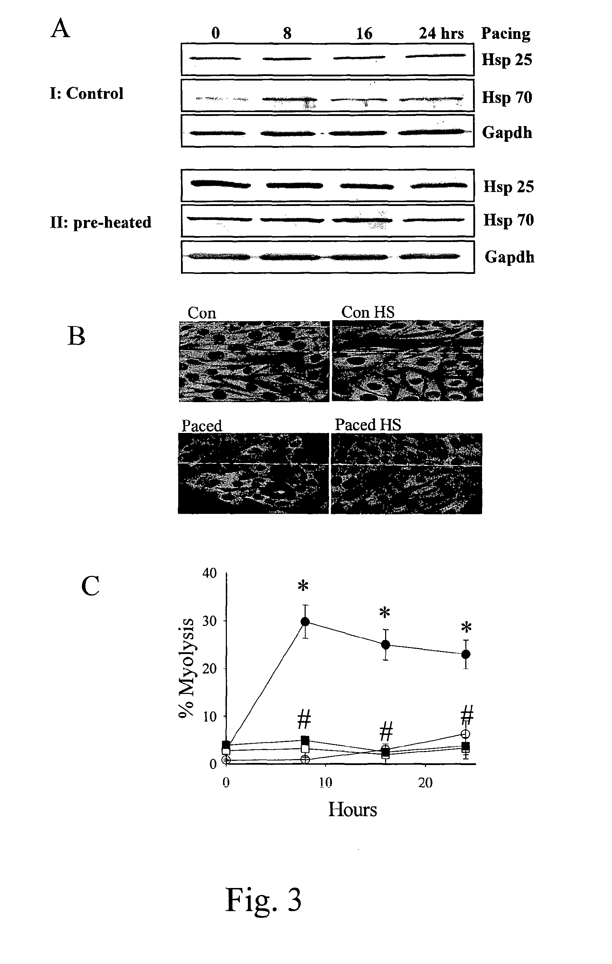

To directly address whether HSP can protect from myolysis induced by AF, we applied a paced cell model for AF which reveals characteristic features of AF.²⁸ This includes the induction of myolysis as seen at 8 hrs of pacing (Figure 3B).

To induce all heat inducible genes, including those encoding HSP25 and HSP70, myocytes were pretreated with a mild non-lethal heat shock and paced from 16 hours afterwards. HSP27 and HSP70 levels were elevated prior to and during pacing (Figure 3A, panel I and II). This heat-shock preconditioning reduced the amount of pacing-induced myolysis (Figure 3B,C).

### HSP27 overexpression is sufficient for protection from pacing-induced myolysis

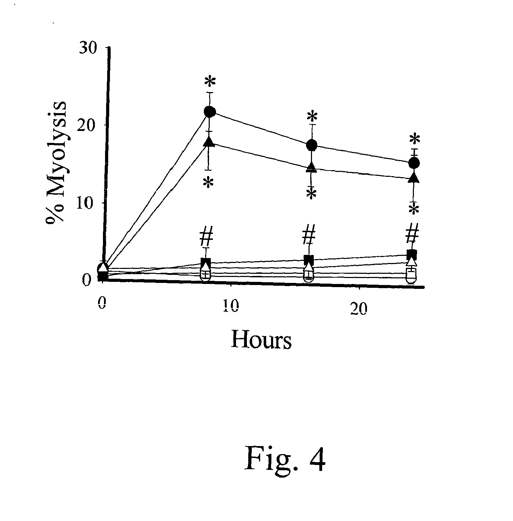

To conclusively establish whether HSP upregulation directly protects from pacing-induced myolysis and to study which HSP conveys this protection, myocytes were transiently transfected with either plasmids encoding HSP70 or HSP27. Myocytes overexpressing HSP27 were protected from pacing-induced myolysis (Figure 4), whereas myocytes overexpressing HSP70 were not (Figure 4). Thus, HSP27 overexpression alone leads to protection from pacing-induced myolysis.

### Discussion

The present disclosure identifies a highly significant increase of protective HSP27 and a somewhat less-profound increase of HSP70 expression in atrial appendages of patients with paroxysmal AF, whereas this upregulation was absent in patients with chronic, persistent AF. The amount of HSP27 and HSP70 correlated inversely with the number of myolytic cells. HSP27 levels also correlated with the duration of chronic AF. Furthermore, HSP27 localized at the myofilaments. Using the HL-1 cell model for AF²⁸, we provided direct evidence that elevated HSP expression prior to pacing attenuates myolysis. Clinically even more relevant, upregulation of HSP during pacing also protected from myolysis. Finally, transfection experiments demonstrated this protection to be attributable to overexpression of HSP27. Elevated HSP expression, and HSP27 in particular, observed in patients with paroxysmal AF are interpreted as an adaptive mechanism to attenuate myolysis resulting in the preservation of myocyte structure and function. Through this mechanism, HSP delays the progression of paroxysmal AF to chronic AF.

### Mechanism of HSP protection

Several mechanisms may explain how HSP27 protect cells from stress-induced damage. The here under provided explanations are not to be constructed to narrow the application. Pacing, directly or via increases of intracellular free calcium and calpain activation^{2;11;28}, might result in protein damage. A first possibility is that HSP27 attenuates AF induced myolysis by their so-called chaperone activity. So far, HSP27 chaperone activity has only been identified in in *vitro* assays in which HSP27 prevented non-native protein aggregation and assisted their refolding.³² In this role, HSP27 alone is not sufficient and depends on cooperation with HSP70.³³ Although we cannot exclude that HSP27 is protective via its presumed chaperone activity, we find this option hard to reconcile since no effect of overexpression of the more potent chaperone HSP70 on pacing-induced myolysis was found. Moreover, using a firefly luciferase technique for measuring protein denaturation³⁴, we found no evidence for pacing-induced protein damage in the HL-1 cell model (Brundel, Schakel and Kampinga unpublished data).

We observed HSP27 to localize at myofilaments in atrial myocytes of AF patients, in line with previous studies in human and rat heart.^{26;35} Therefore, a second and more likely possibility for HSP27 mediated protection is enhanced survival of myocytes following stress by stabilizing of contractile proteins, like tropomyosin, α-actinin and F-actin and/or accelerating their rate of recovery after disruption.^{23;36;37} Since it is known that cystein proteases get activated during AF, and these protease are able to cleave myofilamental proteins^{11;28}, the interaction of HSP27 with contractile proteins may, alternatively, shield them from cleavage by these proteases. Furthermore, the activated cystein proteases also induce apoptosis in certain cells.³⁸ However, when apoptosis is initiated in cardiac myocytes, the activated cystein proteases do usually not cause cell death but rather induce myolysis.³⁹⁻⁴¹ HSP27 was reported to act as anti-apoptotic proteins in several cell types by interfering either with cytochrome c release⁴² or at a later stage during apoptosis, e.g. at the level of protease activity.³⁸ So, as a third option, HSP27 overexpression in myocytes may prevent myolysis by acting in these respective steps of the apoptotic cascade.

### HSP27 expression in paroxysmal AF and progression to chronic AF

In atrial tissue of AF patients, increased HSP27 expression was observed solely in paroxysmal AF. Also pacing induced a temporal, albeit mild induction of HSP25 and HSP70 expression in the cell model for AF. This may be interpreted as early upregulation of HSP during short periods of AF which would enable patients with paroxysmal AF to overcome AF attacks without the induction of structural changes such as myolysis. The most straightforward explanation for the absence of increased HSP expression in chronic AF would be exhaustion of the HSP response as the arrhythmia continues. Exhaustion of HSP upregulation is further supported by the inverse correlation between the duration of chronic AF and the amount of HSP27. Since the heat shock response gets temporarily activated during cardiac differentiation⁴³, disease⁴⁴ and it attenuates with age⁴⁵, one could hypothesize that the exhaustion of the HSP response in time, allows progression from paroxysmal to chronic AF, whereby no protection is present against arrhythmia-induced proteases that lead to myolysis and result in a progressive increase in AF vulnerability.⁴⁶ In this respect, treatment with agents that boost HSP expression during AF may prevent the attenuation of the HSP response and thereby the self-perpetuation of AF.

It needs to be realized that also protective features are ascribed to myolysis. Myolysis is defined as the ability of myocytes to turn into a non functional phenotype, by disruption of the myofibril structure which leads to contractile dysfunction. ^{12;13;20} In general it is believed that myolytic cells do not result in apoptosis but survive prolonged exposure to stress⁴⁷ and thereby form a (secondary) tissue protective response albeit at the loss of cellular function. As such, the heat shock response reflects a first-line defensive mechanism not only maintaining tissue integrity but also tissue function.

In summary, we observed a highly significant increase of protective HSP27 in patients with paroxysmal AF, which correlated with absence of myolysis. This strongly suggests a protective role of HSP27 by attenuating myolysis in these patients. The results obtained from the HL-1 model for AF²⁸, provides direct evidence that elevated expression of HSP27 protects myocytes from pacing-induced myolysis. As such, HSP form an interesting therapeutic target in AF patients to conserve myocyte structure and contractile function.

### DESCRIPTION OF FIGURES

**Figure 1.**
   Protein amounts of HSP70 (A), HSP27 (B) in atrial tissue of patients with paroxysmal AF (PAF), chronic AF (CAF) and controls in sinus rhythm (SR). Protein amounts were determined by Western blotting and expressed as ratios over GAPDH. Inserts show typical Western-blots. Patients with PAF reveal significant increase in HSP70 and HSP27 protein ratios compared to controls in sinus rhythm (SR). (C) Correlation between HSP27/GAPDH protein ratio and duration of CAF.
   *= significant increase compared to SR (p<0.05).
**Figure 2.**
   An inverse correlation was found between the amount of myolysis and protein amounts of HSP70 (A) and HSP27 (B) in patients with PAF (⊙) and CAF (•).
**Figure 3.**
   The effect of induction of HSP levels on pacing induced myolysis. (A) Western blots show that preconditioning by heat shock (HS) induces the expression of HSP70 and HSP27 in time, but do not change GAPDH levels compared to non-treated myocytes (lanes control and 0 hrs). Increased levels are maintained during pacing (lanes 8, 16 and 24 hrs). (B) Immunofluorescent staining of myosin (white) in non paced myocytes (Con) and heat shocked control myocytes (Con HS) compared to 16 hrs paced myocytes (Paced) and paced HS myocytes. Paced myocytes reveal disruption of myosin (myolysis), whereas myosin staining remains diffusely distributed in the cytoplasm of myocytes preconditioned with HS. (C) Quantification of percentage myocytes positive for myolysis in time in control and heat preconditioned myocytes (non-paced myocytes ○, non-paced HS myocytes □, paced myocytes •, paced HS myocytes
   ■ ). *= significant increase compared to non-paced myocytes (p<0.01); #=significant reduction compared to paced myocytes (p≤0.05).
**Figure 4.**
   The effect of HSP27 or HSP70 transfection on pacing-induced myolysis. Quantification of percentage cells positive for myolysis in HSP27 transfeced myocytes (paced HSP27 ■, non-paced HSP27 □), HSP70 transfected myocytes (paced HSP70 A, non-paced HSP70 Δ) compared to untransfected myocytes (paced myocytes •, non-paced control myocytes ○). *= significant increase compared to non-paced control myocytes (p<0.01); #= significant reduction compared to paced control myocytes (p≤0.05).

**Table 1. Baseline characteristics of patients with lone paroxysmal AF (PAF), lone chronic AF (CAF) and control patients in sinus rhythm**

| | | **SR** | **PAF** | **CAF** |
|---|---|---|---|---|
| N | | 8 | 8 | 9 |
| Age | | 61±7 | 51±7 | 54±7 |
| Duration of AF (median, range (months)) | | - | - | 13.4 (0.1-56) |
| Duration SR before surgery (median, range (days)) | | - | 2 (0.5-12) | - |
| Underlying heart disease (n) and /surgical procedure | | | | |
| | Coronary artery disease/CABG | 8 | 0 | 0 |
| | Lone AF Maze | 0 | 8 | 9 |
| New York Heart Association for exercise tolerance | | | | |
| | Class I | 8 | 6 | 5 |
| | Class II | 0 | 2 | 4 |
| Echocardiography | | | | |
| | Left atrial diameter (parasternal) | 37±5 | 40±5 | 45±7 |
| | Left ventricular end-diastolic diameter (mm) | 38±7 | 49±4 | 50±8 |
| | Left ventricular end-systolic diameter (mm) | 29±8 | 38±4 | 30±13 |
| Medication (n) | | | | |
| | Digitalis | 0 | 1 | 5 |
| | Verapamil | 2 | 2 | 4 |
| | Beta-blocker | 4 2 2 | | |
| HSP/Gapdh protein ratio | | | | |
| | HSP27 | 0.8±0.02 | 1.2±0.02* | 0.9±0.03 |
| | HSP40 | 1.5±0.3 | 1.6±0.4 | 1.4±0.3 |
| | Hsc70 | 0.8±0.2 | 0.9±0.3 | 0.7±0.2 |
| | HSP70 | 0.4±0.2 | 1.1±0.3* | 0.8±0.2 |
| | HSP90 | 1.3±0.4 | 1.1±0.5 | 1.2 ±0.4 |

Values are presented as mean value ± SD or number of patients. CABG: Coronary Artery Bypass Grafting; Maze: atrial arrhythmia surgery

### Reference List

1. Godtfredsen J. Etiology, course and prognosis. A follow-up study of 1212 cases. Copenhagen: University of Copenhagen. 1975.
2. Sun H, Chartier D, Leblanc N et al. Intracellular calcium changes and tachycardia-induced contractile dysfunction in canine atrial myocytes. *Cardiovasc Res.* 2001;49:751-761.
3. Ramirez RJ, Nattel S, Courtemanche M. Mathematical analysis of canine atrial action potentials: rate, regional factors and electrical remodeling. *Am J Physiol Heart Circ Physiol.* 2000;279:H1767-H1785.
4. Yue L, Melnyk P, Gaspo et al. Molecular mehanisms underlying ionic remodeling in a dog model of atrial fibrillation. *Circ Res.* 1999;84:776-784.
5. Gaspo R, Sun H, Fareh S et al. Dihydropyridine and beta adrenergic receptor binding in dogs with tachycardia-induced atrial fibrillation. *Cardiovasc Res.* 1999;42:434-442.
6. Brundel BJJM, Van Gelder IC, Henning RH et al. Ion channel remodeling is related to intra-operative atrial refractory periods in patients with paroxysmal and persistent atrial fibrillation. *Circulation.* 2001;103:684-690.
7. Van Wagoner DR, Pond AL, Lamorgese M et al. Atrial L-Type Ca²⁺ Currents and Human Atrial Fibrillation. *Circ Res.* 1999;85:428-436.
8. Liu L, Nattel S. Differing sympathetic and vagal effects on atrial fibrillation in dogs: role of refractoriness heterogeneity. *Am J Physiol.* 1997;273:H805-H816.
9. Wang KKW. Calpain and caspase: can you tell the difference? *TINS.* 2000;23:20-26.
10. Jayachandran JV, Sih HJ, Winkle W et al. Atrial fibrillation produced by prolonged rapid atrial pacing is associated with heterogeneous changes in atrial sympathetic innervation. *Circulation.* 2000;101:1185-1191.
11. Brundel BJJM, Ausma J, Van Gelder IC et al. Activation of Proteolysis by Calpains and Structural Changes in Human Paroxysmal and Persistent Atrial Fibrillation. *Cardiovasc Res.* 2002;54:380-389.
12. Ausma J, Wijffels M, Thone F et al. Structural changes of atrial myocardium due to sustained atrial fibrillation in the goat. *Circulation.* 1997;96:3157-3163.
13. Vanoverschelde JLJ, Wijns W, Depré C et al. Mechanisms of chronic regional postischemic dysfunction in humans: new insights from the study on non-infarcted collateral dependent myocardium. *Circulation.* 1993;87:1513-1523.
14. Manning WJ, Silverman DI, Katz SE et al. Impaired left atrial mechanical function after cardioversion: relation to the duration of atrial fibrillation. *J Am Coll Cardiol.* 1994;23:1535-1540.
15. Leistad E, Aksnes G, Verburg E et al. Atrial contractile dysfunction after short-term atrial fibrillation is reduced by verapamil but increased by BAY K8644. *Circulation.* 1996;93:1747-1754.
16. Allessie M, Ausma J, Schotten U. Electrical, contractile and structural remodeling during atrial fibrillation. *Cardiovasc Res.* 2002;54:230-246.
17. Thijssen VLJL, Ausma J, Liu GS et al. Structural changes of atrial myocardium during chronic atrial fibrillation. *Cardiovasc Pathol.* 2000;9:17-28.
18. Ausma J, Van der Velden HMW, Lenders MH et al. Reverse structural and gap-junctional remodeling after prolonged atrial fibrillation in the goat. *Circulation.* 2003;107:2051-2058.
19. Verheule S, Sato T, Everett T et al. Increased vulnerability to atrial fibrillation in transgenic mice with selective atrial fibrosis caused by overexpression of TGF-b1. *Circ Res.* 2004;94:1458-1465.
20. Allessie MA, Boyden PA, Camm AJ et al. Pathophysiology and prevention of atrial fibrillation. *Circulation.* 2001;103:769-777.
21. Thomas SA., Fallavollita JA, Suzuki G et al. Dissociation of Regional Adaptations to Ischemia and Global Myolysis in an Accelerated Swine Model of Chronic Hibernating Myocardium. *Circ Res.* 2004;91:970-977.
22. Maes A, Flameng W, Nuyts J et al. Histological alterations in chronically hypoperfused myocardium. Correlation with PET findings. *Circulation.* 1994;90:735-745.
23. Bryantsev AL, Loktionova SA, Ilyinskaya OP et al. Distribution, phosphorylation, and activities of HSP25 in heat-stressed H9c2 myoblasts: a functional link to cytoprotection. *Cell Stress Chaperones.* 2002;7:146-155.
24. Lavoie JN, Gingras-Breton G, Tanguay RM et al. Induction of Chinese hamster HSP27 gene expression in mouse cells confers resistance to heat shock. HSP27 stabilization of the microfilament organization. *J Biol Chem.* 1993;268:3420-3429.
25. Van de Klundert FA, Gijsen ML, Van den IJssel PR et al. alpha B-crystallin and HSP25 in neonatal cardiac cells-differences in cellular localization under stress conditions. *Eur J Cell Biol.* 1998;75:38-45.
26. Hoch B, Lutsch G, Schlegel WP et al. HSP25 in isolated perfused rat hearts: localization and response to hyperthermia. *Mol Cell Biochem.* 1996;160-161:231-239.
27. Sanada S, Kitakaze M, Papst PJ et al. Role of phasic dynamism of p38 mitogen-activated protein kinase activation in ischemic preconditioning of the canine heart. *Circ Res.* 2001;88:175-180.
28. Brundel BJJM, Kampinga HH, Henning RH. Calpain Inhibition Prevents Pacing induced Cellular Remodeling in a HL-1 Myocyte Model for Atrial Fibrillation. *Cardiovasc Res.* 2004;62:521-528.
29. Claycomb WC, Lanson NA, Stallworth BS et al. HL-1 cells: A cardiac muscle cell line that contracts and retains phenotypic characteristics of the adult cardiomyocyte. *Proc Natl Acad Sci USA.* 1998;95:2979-2984.
30. Hirakawa T, Rokutan K, Nikawa T et al. Geranylgeranylacetone induces heat shock proteins in cultured guinea pig gastric mucosal cells and rat gastric mucosa. *Gastroenterology.* 1996;111:345-357.
32. Jakob U, Gaestel M, Engel K et al. Small heat shock proteins are molecular chaperones. *J Biol Chem.* 1993;268:1517-1520.
33. Ehrnsperger M, Graber S, Gaestel M et al. Binding of non-native protein to HSP25 during heat shock creates a reservoir of folding intermediates for reactivation. *EMBO J.* 1997;16:229.
34. Michels AA, Kanon B, Konings AW et al. HSP70 and HSP40 chaperone activities in the cytoplasm and the nucleus of mammalian cells. *J Biol Chem.* 1997;272:33283-33289.
35. Lutsch G, Vetter R, Offhauss U et al. Abundance and location of the small heat shock proteins HSP25 and aB-Crystallin in rat and human heart. *Circulation.* 1997;96:3466-3476.
36. Somara S, Bitar KN. Tropomyosin interacts with phosphorylated HSP27 in agonist-induced contraction of smooth muscle. *Am J Physiol Cell Physiol*. 2004;286:C1290-C1301.
37. Lavoie JN, Lambert H, Hickey E et al. Modulation of cellular thermoresistance and actin filament stability accompanies phosphorylation-induced changes in the oligomeric structure of heat shock protein 27. *Mol Cell Biol*. 1995;15:505-516.
38. Leist M, Jäättelä M. Four deaths and a funeral: from caspases to alternative mechanisms. *Nat Rev Mol Cell Biol.* 2001;2:589-598.
39. Communal C, Sumandea M, De Tombe P et al. Functional consequences of caspase activation in cardiac myocytes. *Proc Natl Acad Sci USA.* 2002;99:6252-6256.
40. Moretti A, Weig HJ, Ott T et al. Essential myosin light chain as a target for caspase-3 in failing myocardium. *Proc Natl Acad Sci USA.* 2002;99:11860-11865.
41. Du J, Wang X, Miereles C et al. Activation of caspase-3 is an initial step triggering accelerated muscle proteolysis in catabolic conditions. *J Clin Invest.* 2004;113:115-123.
42. Pandey P, Farber R, Nakazawa A et al. HSP27 functions as a negative regulator of cytochrome c-dependent activation of procaspase-3. *Oncogene.* 2000;19:1975-1981.
43. Benjamin IJ, Shelton J, Garry DJ et al. Temporospatial expression of the small HSP/alpha B-crystallin in cardiac and skeletal muscle during mouse development. *Dev Dyn.* 1997;208:75-84.
44. Aufricht C, Ardito T, Thulin G et al. Heat-shock protein 25 induction and redistribution during actin reorganization after renal ischemia. *Am J Physiol.* 1998;274:F215-F222.
45. Demirel HA, Hamilton KL, Shanely RA et al. Age and attenuation of exercise-induced myocardial HSP72 accumulation. *Am J Physiol Heart Circ Physiol.* 2003;285:H1609-H1615.
46. Todd DM, Fynn SP, Walden AP et al. Repetitive 4-week periods of atrial electrical remodeling promote stability of atrial fibrillation: time course of a second factor involved in the self-perpetuation of atrial fibrillation. *Circulation.* 2004;109:1434-1439.
47. Dispersyn GD, Ausma J, Thone F et al. Cardiomyocyte remodeling during myocardial hibernation and atrial fibrillation: prelude to apoptotis? *Cardiovasc Res.* 1999;43:947-957.

## Claims

1. A method for at least in part preventing or delaying or decreasing damage to a cardiac cell induced by a supraventricular arrhythmia comprising increasing the amount of at least one heat shock protein (HSP) or a functional equivalent and/or a functional fragment thereof in said cardiac cell.

2. A method according to claim 1 wherein said HSP is HSP27 or an HSP27-like protein or a functional equivalent and/or a functional fragment thereof.

3. A method according to claim 1 or 2 wherein said HSP is increased in said cell by transfecting said cell with a gene encoding said HSP or a functional equivalent and/or a functional fragment thereof.

4. A method according to claim 1 or 2 wherein said HSP is increased in said cell by injecting into said cell an HSP protein or a functional equivalent and/or a functional fragment thereof.

5. A method according to claim 1 or 2 wherein said HSP is increased by providing said cell with a drug.

6. A method according to claim 1 or 2 wherein said HSP is increased by heat preconditioning of said cell.

7. A method according to any one of claims 1 to 6 wherein said supraventricular arrhythmia is atrial fibrillation.

8. A method according to any one of claims 1 to 7 wherein said cell is a myocyte.

9. A method according to any one of claims 1 to 8 wherein said damage is myocyte remodeling.

10. A method according to claim 9 wherein said myocyte remodeling is myolysis.

11. A method according to any one of claims 1 to 10 which is performed in *vitro.*

12. A pharmaceutical composition comprising at least one nucleic acid encoding HSP or a functional equivalent and/or a functional fragment thereof and/or comprising at least one HSP protein or a functional equivalent and/or a functional fragment thereof and further comprising a pharmaceutical acceptable carrier or diluent.

13. A pharmaceutical according to claim 12 wherein said HSP is HSP27 or an HSP27-like protein or a functional equivalent and/or a functional fragment thereof.

14. A pharmaceutical according to claim 12 or 13 wherein said nucleic acid encoding HSP or a functional equivalent and/or a functional fragment thereof is part of a gene delivery vehicle.

15. Use of at least one gene encoding an HSP protein or a functional equivalent and/or a functional fragment thereof or at least one HSP protein or a functional equivalent and/or a functional fragment thereof for the (in *vitro)* treatment of a supraventricular arrhythmia.

16. Use of at least one nucleic acid encoding an HSP protein or a functional equivalent and/or a functional fragment thereof or at least one HSP protein or a functional equivalent and/or a functional fragment thereof for the manufacture of a medicament for the treatment of a supraventricular arrhythmia.

17. Use according to claim 16 wherein said HSP is HSP27 or an HSP27-like protein or a functional equivalent and/or a functional fragment thereof.

18. Use according to claim 16 or 17 wherein said nucleic acid encoding an HSP protein or a functional equivalent and/or a functional fragment thereof is part of a gene delivery vehicle.

19. Use according to any one of claims 14 to 19 wherein said supraventricular arrhythmia is atrial fibrillation.
